# EUROPEAN PATENT APPLICATION

(11) **EP 4 349 264 A1**
(43) Date of publication of application: **10.04.2024**
(21) Application number: 22315229.9
(22) Date of filing: 05.10.2022
(51) Int. Cl.: A61B 8/12, A61B 8/00, A61B 8/08

(54) **MEDICAL DEVICE AND SYSTEM COMPRISING A MEDICAL DEVICE**

(71) Applicant: Caranx Medical SAS, 75013 Paris (FR)
(72) Inventor: BERTHET-RAYNE, Pierre, 06800 Cagnes-sur-Mer (FR); MIRA, Anna, 06000 Nice (FR); SMITS, Hamen Victor Jonas, 3360 Bierbeek (BE); CERRUTI, Giulio, 06700 Saint-Laurent-Du-Var (FR); PIETERS DAMERIJAN, Vera, 06140 Vence (FR); MIRCEA, Moscu, 06100 Nice (FR); BECHAR, Ikhlef, 06410 Biot (FR); PRZYBYLSKI, Flavie, 06800 Cagnes sur Mer (FR)
(74) Representative: Hepp Wenger Ryffel AG

(57) **Abstract**

The invention relates to a medical device (101, 103) for use with or as a part of an elongated medical catheter (201), preferably during transcatheter valve implantation. The device (101, 103) comprises at least one imaging probe (1), in particular an ultrasound probe, for local imaging of a body lumen (11) of a patient (12) and a shape sensing unit (2) for detecting a partial or complete shape (13) of the device (101, 103). The imaging probe (1) is arranged neighbouring a distal end of the device (101, 103) and in such a way that a field of view (4) of the imaging probe (1) is or can be arranged such as to image an area proximal to the distal end, preferably by the field of view (4) being directed in a proximal direction (15) of the device (101, 103).

## Description

The invention relates to a medical device for use with or as a part of an elongated medical catheter and a system comprising a medical device according to the independent claims.

Insertion of elongated medical catheters into and through a body lumen during minimally invasive procedures, in particular during transcatheter valve implantation, bears the risk of surgical complications, i.e. tissue damage, bleeding, arrythmias, heart attack, stroke and even vessel rupture. Moreover, this risk is further increased by patient movement and/or deformation of the body lumen, in particular during the systole/diastole cycle.

In addition, contact with a wall of the body lumen, such as a blood vessel, in particular an artery, can cause injury to the wall or dislodge deposits such as e.g. calcification, causing potentially life-threatening complications to the patient such as ischemic strokes.

It is common for interventional fluoroscopy to be used to track a medical elongated catheter, e.g. during transcatheter aortic valve implantation. However, fluoroscopy carries harmful X-rays for both the patient and the surgeon. Therefore, recent technological advances focus on a less invasive use of ultrasound probes, in particular ultrasound probes mounted on a medical catheter.

EP 2 068 716 A1 discloses an interventional device comprising an ultrasound transducer adapted to acquire flow data and an ultrasound probe for acquiring positioning data.

US 9,404,734 B2 discloses an instrument system and method for sensing shape, position, and orientation of an elongate body based on a strain sensor and localization sensor.

Vander Poorten, E., Tran, P., Devreker, A., Gruijthuijsen, C., Portoles-Diez, S., Smoljkic, G., ... Herijgers, P. (2016). Cognitive autonomous CAtheters Operating in Dynamic Environments. Journal of Med\ca\ Robotics Research, 01(03), 1640011. doi:10.1142/s2424905x16400110 discloses an Intra Vascular Ultra Sound (IVUS) catheter which enables intra-operative vascular reconstruction and is provided with multiple electromagnetic tracking (EMT) sensors for measuring a pose and position of the catheter.

However, there is generally a lack of a medical device which allows for local imaging of a body lumen sufficient for intra-operative three-dimensional imaging of a constantly moving/deforming body lumen, in particular at the region of the heart valve, and allows for precise, reliable, and safe positioning of a medical catheter with respect to the body lumen.

It is an object of the present invention to overcome these and other disadvantages of the prior art. In particular, the invention shall provide a medical device which provides improved imaging of the body lumen by detecting time-dependent intraluminal deformations of the body lumen.

In addition, the medical device shall enable a precise localization of the partial or complete shape of the medical device with respect to the body lumen such that the positioning and deformation of the medical catheter can be optimized.

Furthermore, the invention shall provide imaging of the body lumen which allows for identifying vessel narrowing or dilation, calcifications, potential aneurisms or stenotic areas.

It is further an object of the present invention to provide a medical device which may be used with existing medical elongated catheters known by the prior art, so that they can be easily retrofitted with the medical device.

The objects are solved by a medical device and system comprising a medical device as defined in the independent claims. Further embodiments result from the dependent claims.

A medical device for use with or as a part of an elongated medical catheter, preferably during transcatheter valve implantation, according to the invention comprises at least one imaging probe, in particular an ultrasound probe, for local imaging of a body lumen of a patient. The medical device further comprises a shape sensing unit for detecting a partial or complete shape of the device. The imaging probe is arranged neighbouring a distal end of the device in such a way that a field of view of the imaging probe is or can be arranged such as to image an area proximal to the distal end, preferably by the field of view being directed in a proximal direction of the device.

The field of view is defined as a spatial region in which a local image can be acquired by using the imaging probe, e.g. based on a divergence of a transducer array, a transducer array size of an ultrasound probe, and an ultrasound imaging depth.

In this context, the proximal and distal directions are defined as the proximal direction toward an operator of the device and the distal direction away from the operator.

Arranging the field of view of the imaging device in a proximal direction can allow for retrograde imaging e.g. of a heart valve/annulus from within the heart ventricle, such that the crossing and/or positioning of the device and preferably the medical catheter and a valve prosthesis mounted on the catheter relative to the heart valve, preferably considering the cardiac cycle of the patient, can be observed/monitored.

The imaging probe being an ultrasound probe can comprise a sector array, linear array, or curved array ultrasound transducer. However, the ultrasound does not need to penetrate deep into the tissue since local imaging of the wall of the body lumen is sufficient. Moreover, a decreasing lateral resolution based on a diverging ultrasound in contrast to linear array transducers is not an issue. Therefore, sector array or curved array ultrasound transducers are preferred for the device.

A longitudinal field of view along a longitudinal axis, in particular along a proximal or distal direction, of the device can have an angulation of 90°, in particular 60°, more preferably 35°.

The imaging probe can have a sampling rate of less than 750 ms, in particular less than 250 ms, more preferably less than 100 ms. A faster sampling rate increases the amount of data which has to be processed but allows for a faster, preferably near real-time, adjustments of the device, e.g. based on a processing unit.

The device can have a total length of 160 cm, in particular 120 cm, preferably 90 cm. Preferably, the length of the device is such that transcatheter heart valve implantation can be performed using the device.

The shape sensing unit can comprise an optical fiber, in particular a plurality of optical fibers, configured for detecting the shape of the device based on at least one fiber Bragg grating. An implementation of an optical fiber comprising fiber Bragg gratings into an elongated medical device is described extensively in the prior art document US 9,404,734 B2.

The optical fiber can comprise a uniform fiber Bragg grating, an apodized fiber Bragg grating, in particular Gaussian or raised-cosine fiber Bragg grating, a chirped fiber Bragg grating, a tilted fiber Bragg grating, and/or a superstructure fiber Bragg grating.

The different fiber Bragg grating can be used for optimizing the shape reconstruction, e.g. the apodized fiber Bragg grating can allow for wavelength dependent dispersion and broadening of a reflective spectrum.

The plurality of optical fibers, in particular a parallel plurality of optical fibers, can be arranged along the longitudinal direction of the device, a circumferential direction of the device or a helical direction of the device.

The plurality of optical fibers can be arranged radially spaced apart from a central longitudinal axis of the device. The optical fibers may be arranged at different circumferential positions of the device, in particular at equidistant circumferential positions of the device.

The device may comprise a channel for insertion of the at least one optical fiber relative to the device, in particular a plurality of channels for insertion of multiple optical fibers independently from each other.

The optical fiber may comprise a measuring segment which comprises the fiber Bragg grating(s) which only extends along a longitudinal partial section of the optical fiber. In particular, the device may comprise multiple measuring segments which extend along multiple longitudinal partial sections of the optical fiber.

The shape sensing unit can comprise a reflecting element which is configured for at least partially reflecting light non-wavelength-dependent within an optical fiber.

The reflecting element can be configured to reflect the light along a direction of incidence to an opposite direction essentially completely, in particular a longitudinal direction of the optical fiber. The reflecting element can be arranged at the distal end of the device, in particular at a terminal end of the distal end.

Alternatively, the shape sensing unit may comprise at least one, in particular a plurality, of electromagnetic field sensors.

The device can be insertable into the elongated medical catheter, into the body lumen, e.g. the aorta or the heart ventricle, or a branching body lumen, e.g. a carotid or coronary artery.

This allows for easily retrofitting of existing medical elongated catheters with the device. In addition, the device can be inserted independently from the elongated catheter into the body lumen and/or branching body lumen. Thereby, the medical device can be used for imaging of a body lumen, the branching body lumen and/or detecting a partial or complete shape of the device independently from the medical catheter. The advancement of the elongated catheter, which often times has a larger diameter than the medical device, can subsequently be carried at a lower risk to the patient because additional data is obtained by the prior insertion and/or image acquisition of the medical device.

The device may form a medical catheter, or the device may be used as a guidewire for guiding the medical catheter. The device may have a closed distal end without any openings.

The device can for example be advanceable across the aortic valve annulus into a left ventricle of a patient, prior to the introduction of a medical catheter. The imaging probe neighbouring the distal end of the device can be arranged such as to image an area proximal to the distal end, e.g. sections of the aortic valve annulus can be imaged which are not easily assessable, at least not in detail, from a position proximal the aortic valve annulus. The position proximal the aortic valve is still defined from the perspective of a clinician and not with respect to the aortic valve annulus.

The device being insertible into the elongated catheter can have a cross-sectional diameter of 3 mm, in particular less than 2 mm, preferably less than 1 mm. A small cross-sectional diameter of the device allows for a safer insertion into the body lumen and deflection of the device in accordance with natural bends of the body lumen.

An outer surface of the medical device may be coated with an anti-friction and/or hydrophobic coating, in particular polytetrafluoroethylene and/or fluoroethylene propylene and/or silicones.

The device can be advanceable over the elongated medical catheter, in particular by the device being evertible in a longitudinal direction of the elongated medical catheter.

An evertible device has an inner portion and an outer portion. The inner portion is configured to be everted into the outer portion at a distal end of the device, in particular by moving an everting member and/or by applying a pressure to an inner chamber formed between the inner and outer portion.

The evertible device allows that the outer portion is essentially immovable with respect to the wall of the body lumen and only "grows" by everting the inner portion such that friction between the device and the wall can be minimized even if the outer portion contacts the wall.

The inner portion of the evertible device can be configured to be positioned directly adjacent an outer surface of the medical catheter. The radial distance between the inner portion and the outer portion can be less than 1.5 mm, in particular less than 1 mm, preferably less than 0.5 mm.

The device can comprise a deflecting mechanism for partially or fully deforming the device and in particular the elongated medical catheter.

The deflecting mechanism allows for deflecting the device and/or catheter based on the imaged body lumen and shape of the device such that the device and in particular the medical catheter can be advanced in the body lumen without contacting the wall of the body lumen.

Furthermore, for example, a non-steerable, elongated medical catheter into which the device is inserted can be made steerable by actuating the device's deflecting mechanism.

The deflecting mechanism can comprise or consist of a shape memory alloy/metal, comprise piezoelectric elements, and/or push rods for actuating the deflecting mechanism.

The deflecting mechanism can comprise at least one deflecting element, in particular a tendon and/or pull wire, for deforming a deflecting section.

The deflecting section may comprise at least two, preferably at least three deflecting elements. The deflecting elements can be arranged to allow for movement of the device, and in particular the medical catheter, along two degrees of freedom.

The deflecting section can be configured to be adjustable in length in the longitudinal direction of the device, e.g. by moving the deflecting section with respect to a stiff section of the device.

The device may comprise multiple longitudinally extending deflecting sections which are deflectable independently from each other by the deflecting mechanism or separate deflecting mechanisms.

The plurality of deflecting sections may be telescopically, in particular concentrically, arranged with respect to each other.

The device can comprise a plurality of imaging probes. The' field of view of one of the imaging probes is preferably oriented along a distal or radial direction and the field of view of one other imaging probe is oriented along a proximal direction of the device.

The device comprising multiple imaging probes allows to reconstruct a more complete and higher resolution model of the body lumen for determining the position of the device and/or of obstructions of the body lumen. In addition, a proximally and distally oriented imaging probe allows for optimized axial movement in the distal as well as the proximal direction of the device.

The imaging probe with a field of view which is oriented along the proximal direction and/or the imaging probe which is oriented along the distal direction can be immovably mounted neighbouring the distal end. Thus, the imaging probe(s) can have no unnecessary moving parts and are less prone to malfunction or defects.

Alternatively or additionally, the positioning unit can be adapted for pivoting the imaging probe such as to tilt its field of view relative to the longitudinal axis of the device.

Alternatively or additionally, the positioning unit can be adapted for pivoting a plurality of imaging probes such as to tilt their respective field of view relative to the longitudinal axis, preferably simultaneously.

Thereby, at least one imaging probe can be reversibly movable from a distally-looking configuration, having a field of view oriented along the distal direction, to a proximally-looking configuration, having a field of view oriented along the proximal direction of the device.

The imaging probe being convertible between the distally-looking and the proximally-looking configuration allows for a more versatile imaging capability of the body lumen, in particular without necessarily requiring a plurality of imaging probes.

In addition, one or more such imaging probes allow to scan an area along a slope relative to the longitudinal axis of the device, providing more detailed and complete imaging, e.g. for reconstructing a three-dimensional model of the body lumen. The device with multiple imaging probes further allows a faster imaging procedure.

The plurality of imaging probes can be spaced apart from each other in a circumferential and/or longitudinal direction of the device, in particular equidistantly spaced apart from each other. If the distance between the imaging probes is fixed or fixable, the positions of the imaging probes with respect to each other do not change and the reconstructed image of the body lumen can be enhanced, in particular if the body lumen moves/is deformed.

The imaging device may be arranged on a longitudinal extension and the positioning device can be configured to tilt or rotate the longitudinal extension with respect to the device. A plurality of imaging devices on the longitudinal extension can be arranged in an umbrella shape, in particular on the distal end of the device.

The positioning device can be configured such that the imaging probe(s) are movable together, in particular exclusively movable together. Thereby, the images can be captured by the imaging sensor(s) simultaneously at a given time. Thus, for example, different areas at a specific tilt of the imaging probe/longitudinal extension with respect to the longitudinal axis of the device can be recorded simultaneously. Consequently, the recorded images for reconstructing the three-dimensional shape do not have to be corrected for individual positions of the imaging probe with respect to the body lumen at the given time the image was acquired.

The imaging probe(s) can be movable in an axial direction of the device. An imaging probe which is movable relative to the longitudinal axis of the device allows for changing the field of view of the imaging probe even when the device as such is at a fixed position.

The positioning unit can be bistable such that it is only in a stable position if the field of view is oriented in the distally-looking configuration or the proximally-looking configuration. This ensures that the position of the imaging probe is fixed when the positioning unit is not actively actuated to change the field of view, and that the optimal alignment position of the imaging probe is precisely adopted.

The device can comprise a contact force sensor neighbouring the distal end of the device.

A contact force sensor allows for detection if the distal end of the device contacts the wall of the lumen body.

In addition or alternative to the contact force sensor, the distal end of the device can comprise a proximity sensor, in particular a proximity sensor from the group of a fiber Bragg grating sensor, an electromagnetic proximity sensor, and ultrasound proximity sensor or an electric force sensor, preferably a resistive force sensor.

The device can comprise at least one pressure sensor for measuring a local pressure of a body lumen external to the device, in particular at least two pressure sensors which are arranged spaced apart from each other, in particular spaced apart from each other in the longitudinal direction of the device.

The pressure sensor allows for measuring a pressure indicative of the cardiac cycle. Furthermore, the pressure sensor allows for detecting the position/point in time of crossing the valve annulus based on a pressure gradient between an artery and the left heart ventricle.

Two pressure sensors which are spaced apart in longitudinal direction of the device allow optimized positioning of the device and the medical catheter, e.g. for crossing the artery valve annulus.

The pressure sensor may be arranged at the distal end of the device.

The device can comprise at least one channel leading from a measurement site to the pressure sensor, in particular at least two channels leading from a respective measurement site to a respective pressure sensor or a common pressure sensor.

The channel(s) leading to the pressure sensor(s) enable(s) the pressure sensor to be placed at an arbitrary position of the device.

In addition, a plurality of channels leading to one pressure sensor allow for only one pressure sensor to be used for measuring the pressure at a plurality of measurement sites located at an end of the channel opposite the pressure sensor.

The at least one pressure sensor can be configured for measuring the static pressure at a circumferential section of the device and/or the total pressure consisting of a dynamic and the static pressure, e.g. at a front side of the distal end of the device.

The pressure sensor can be arranged partially, preferably completely, within the device.

Measuring the pressure with a pressure sensor at a particular circumferential section of the device and/or the total pressure provides additional information of the blood flow at a particular position of the device.

In addition, a pressure sensor can allow for determining constrictions within the body lumen which result in an increased blood flow and therefore dynamic pressure.

Furthermore, the pressure sensor can detect pressures indicative of the length and/or cross-sectional area of e.g. a constriction within the body lumen, e.g. determined deterministically by the Hagen-Poiseuille law or a machine learning algorithm.

The device can comprise a flow sensor for detection of the blood flow. The flow sensor can be positioned at a distal end of the device.

The flow sensor and/or a pressure sensor for measuring the complete pressure, in particular in conjunction with a pressure sensor for only measuring the static pressure, can be used to determine a radial position of the device with respect to the body lumen. The flow, e.g. blood flow, within the body lumen, e.g. the artery, in general has a parabolic velocity profile based on the boundary conditions at the walls of the body lumen. Therefore, the velocity of blood flow determined based on a flow sensor, a dynamic pressure, and/or the complete pressure, allows for determining the radial position within the body lumen, e.g. by using a processing unit.

The device can comprise at least one sensing element which is selected from the group of a conductivity sensor and a bio-impedance sensor.

The conductivity sensor and/or bio-impedance sensor can be used for classifying tissue by measuring the electrical conductivity. The tissue classification can be used, for example, to optimize the placement of a prosthetic heart valve.

According to another aspect, a medical device, optionally a previously described medical device, in particular for use with an elongated medical catheter during transcatheter valve implantation according to the invention comprises at least one imaging probe, in particular an ultrasound probe, for local imaging of a body lumen of a patient. The device further comprises a shape sensing unit for detecting a partial or a complete shape of the device. The device comprises at least two pressure sensors which are spaced apart from each other or movable relative to each other, in particular in a longitudinal direction of the device. The pressure sensors are configured for detecting a pressure gradient within the body lumen.

The two pressure sensors can be movable relative to each other in radial, circumferential and/or longitudinal direction of the device which allows for registration of pressures, in particular dynamic and static pressures, to spatial positions. Based on the registered pressure changes, especially in longitudinal direction of the body lumen, reconstruction of a pressure map, a velocity map and/or a blood flow distribution is possible, e.g. by the processing unit.

A system according to still another aspect of the invention comprises a medical device, preferably a previously described medical device, in particular for use with an elongated medical catheter during transcatheter valve implantation. The device has a shape sensing unit for detecting a partial or complete shape of the device. The device further has at least one imaging probe for local imaging of a body lumen of a patient, which is arranged neighbouring a distal end of the device and/or multiple spaced apart pressure sensors located on the device or insertable into the device. The imaging probe can be an ultrasound probe. The system further has the processing unit connected or connectable to the shape sensing unit and the imaging probe and/or the pressure sensors.

The processing unit is configured for processing data relating to a local image of the body lumen acquired in a distally-looking configuration having a field of view oriented in a distal direction of the imaging probe and/or acquired by the imaging probe in a proximally-looking configuration having a field of view oriented in the proximal direction.

Alternatively or additionally, the processing unit is configured for processing data relating to the partial or complete shape of the device acquired by the shape sensing unit.

Alternatively or additionally, the processing unit is configured for processing data relating to patient-specific pre-operative three-dimensional data, in particular pre-operative computer tomography data.

Alternatively or additionally, the processing unit is configured for processing data relating to a detected signal of a contact force sensor and/or a detected signal, in particular detected signals, of the pressure sensor.

The processing unit is further configured to determine, based on the processed data, at least one operating signal for operating at least one of a deflecting mechanism of the device, a positioning unit for positioning the imaging probe and an advancement actuator for advancing the device.

The system having a processing unit determining an operating signal enables autonomous operability of the device and preferably the medical catheter. Thus, a surgical intervention using the system, e.g. a heart valve replacement, can be simplified and accelerated partly or fully autonomously and further is independent/less dependent on the skill of the clinician.

The system can comprise the medical elongated catheter. The device may be immovably arranged within the medical catheter or movable within a lumen of the medical catheter. The medical catheter may comprise multiple lumens.

The medical catheter of the system can have a maximum outer diameter of 16 Fr, in particular 11 Fr, preferably 6 Fr.

An inner lumen of the medical catheter can have a diameter of 2 mm, in particular 1.5 mm, in particular preferably 1 mm.

The lumen of the medical catheter may comprise a proximal opening and a distal and/or lateral opening for guiding the medical device distally or laterally into the body lumen and/or a branching body lumen.

The processing unit can be adapted for determining a cardiac cycle of a patient based on the processed data. The processing unit can further be adapted for operating at least one of the deflecting mechanism, the positioning device, and the advancement actuator based on the determined cardiac cycle. Thereby, the device and/or the medical catheter can e.g. be introduced via a heart annulus into the left ventricle of the heart coordinated or synchronized with the cardiac cycle.

The processing unit can be adapted to determine the position of the device based on the processed data, in particular the radial position of the device, in particular the distal end of the device, with respect to the body lumen.

The processing unit can be adapted to determine, based on the processed data, a length in the longitudinal direction of the body lumen, and/or a cross-sectional area of a constriction/obstruction of the body lumen.

The processing unit can be adapted to determine, based on the processed data, a pressure and/or velocity distribution of the blood, preferably along the longitudinal direction of the body lumen, or along the radial direction of the body lumen.

The processing unit can be adapted to determine, based on the processed data, the cardiac/respiratory cycle and corresponding spatial changes of the body lumen. The processing unit can be adapted to adjust the operating signal(s) accordingly in antici- , pation of predicted future spatial changes of the body lumen based on the determined cardiac/respiratory cycle and/or spatial changes.

The processing unit can be configured to carry out a volume scan by operating the positioning unit to move the imaging probe from the distally-looking configuration to the proximally-looking configuration or vice versa. The processing unit can be configured to reconstruct or augment a three-dimensional model based on the volume scan, in particular a model of the left ventricle of a heart. The processing unit can further be configured to determine, based on the three-dimensional model, at least one operating signal for operating at least one of the deflecting mechanism of the device, the positioning unit, and the advancement actuator for advancing the device.

The processing unit can be configured such that the imaging probe(s) acquire local images at predetermined angular intervals of the imaging probe or at a specific sampling rate throughout the volume scan.

The volume scan can extend only over a partial angular range by adjusting the positioning unit, e.g. move less than 180°, in particular less than 120°, preferably less than 75° between the proximally looking and distally-looking configuration.

During a transcatheter aortic valve implantation, a guidewire is often inserted into the valve annulus to guide the medical catheter across the valve annulus for valve implantation.

The device can be configured, analogously to a guidewire, to guide the medical catheter and be inserted into the valve annulus prior to the medical catheter, e.g. from a distal opening of the lumen within the medical catheter. Subsequently, a volume scan can be performed. The reconstructed three-dimensional model based on the volume scan allows for subsequent precise positioning and crossing of valve annulus of the medical catheter, e.g. into the left ventricle. Thus, the shape of the inner surface of the valve annulus and in particular the spatial position and distribution of calcifications can be determined in subsequent steps.

The processing unit can be adapted to carry out the volume scan more than once and/or based on a specific point in time, e.g. based on or triggered by the cardiac cycle, in particular at a specific point in time during a diastole or a systole phase.

The processing unit can be adapted to extend the three-dimensional model to a four-dimensional model based on the time-dependent three-dimensional deformations of the body lumen, in particular based on the cardiac cycle and/or on the respiratory cycle.

The processing unit can be further configured to adapt the three-dimensional model to include tissue properties, in particular mechanical tissue properties, e.g. the flexural mechanical properties of heart valves or flaps/cusps. The tissue properties may be determined based on reference values which are converted by the processing unit. Alternatively, the processing unit can be configured to determine the tissue properties, for example by a conductivity sensor, bio-impedance sensor or based on a structural position/movement of the tissue on different local images.

Including tissue properties in the three-dimensional model allows for optimized spatial placement and fixation of an implant, in particular a heart valve prosthesis, and minimizing the risk of injury to the patient.

The processing unit can be configured to process the local images of the imaging probe to determine a blood flow in a local section of the body lumen.

The blood flow in a local section of the body lumen can be reconstructable by the processing unit based on a plurality of acquired local images of the imaging probe, in particular the ultrasound probe, using Doppler ultrasonic imaging.

The processing unit can be adapted to reconstruct landmarks in the three-dimensional model, e.g. indicative of points of interest, such as particular positions of the heat valve annulus, flaps/cusps, calcifications and/or obstructions/constrictions.

The processing unit can be adapted to overlay a virtual model of a prosthetic heart valve with a virtual three-dimensional model of an area of a patient, preferably the reconstructed three-dimensional model, in particular by using landmarks in the reconstructed three-dimensional model. Alternatively, the processing unit can be adapted to overlay the virtual model of a prosthetic heart valve with a virtual model of an area of a patient by using a machine learning algorithm based on the reconstructed three-dimensional model. The processing unit may further be adapted to display the overlayed virtual models on a display.

The processing unit can be adapted to register images, in particular the local images of the proximally-looking configuration and/or the reconstructed three-dimensional model, with pre-operative three-dimensional data, in particular to identify calcification locations based on the pre-operative three-dimensional data.

The registration of images and/or the reconstructed three-dimensional model with pre-operative three-dimensional data or time-dependent pre-operative three-dimensional data allows for a more accurate determination of the position of the device and/or medical catheter with respect to the body lumen. In addition, estimated or approximated data indicative of sections of the body lumen which have not been imaged by the imaging probe can be generated by the processing unit.

Typically, certain obstructions/constrictions and/or materials, e.g. calcifications, are not or only poorly visible by imaging probes, in particular an ultrasound probe. Therefore, registration of the three-dimensional model or images to pre-operative three-dimensional data, e.g. from a computer tomography, can allow for better determining the locations of these obstructions/constrictions and/or materials within the three-dimensional model or images.

The system can comprise a memory unit connected or connectable to the processing unit for storing data of pre-operative data, the reconstructed three-dimensional model or the overlayed virtual models, the local blood flow, the detected signal of the contact force sensor, and/or the detected signal of the at least one pressure sensor.

The memory unit allows to retrieve already determined/measured data in order to optimize the control of the device by the processing unit.

The processing unit can be adapted for verifying a position of a prosthetic heart valve within the heart based on the stored data of the memory unit prior to a placement of the prosthetic heart valve by comparison to processed data after the placement of the prosthetic heart valve.

Reliably verifying the position and optionally repositioning the prosthetic heart valve, if necessary, minimizes the long-term health risks. In addition, the processing unit can be adapted to detect regurgitation or the risk of future regurgitation of the prosthetic heart valve based on the processed data.

The verification of the placement can comprise leak detection by (i) local images of the imaging probe prior and after implantation, (ii) pre-operative volume data, (iii) overlayed virtual models verification, (iv) valve fitting evaluation, (v) pressure gradients and/or (vi) velocity distribution of the blood flow. The detection of leakage and the velocity distribution of the blood flow can e.g. be determined by a Doppler ultrasound of the ultrasound probe.

The processing unit can be adapted to operate the deflecting mechanism and/or the advancement actuator based on the processed data and in particular on at least one of the stored data. Thereby, a distal end of the device, in particular the complete device, preferably the medical catheter, typically can be controlled to remain centred within the body lumen at least partially along its length.

Centering the device and preferably the medical catheter within the body lumen minimizes the risk of trauma on vessel walls or dislodging calcifications. By taking into account stored data, centric positioning can further be optimized, since e.g. stored data concerning the measured blood flow at a different location can contribute to optimization at a current location of the device/medical catheter.

The processing unit can be adapted for operating the device during a transcatheter valve implantation in a fully automated manner, in particular based on a reinforcement learning algorithm.

The system, in particular the device", can comprise a pacing member for rapid pacing.

Rapid pacing allows to temporarily reduce the ventricular output during transcatheter valve implantation to ensure transient cardiac standstill, e.g. while pre-dilatation is performed and the prosthetic heart valve is being positioned and deployed.

The processing unit can be adapted to adjust the partial or complete shape of the device via the deflecting mechanism when-advancing the device in the body lumen via the advancement actuator based on the spatial position and orientation of the distal end of the device with respect to the body lumen. Thereby, subsequent portions of the device adopt the same spatial position and orientation with respect to the body lumen.

This "follow the lead steerability" (e.g. as disclosed in WO 2021/216782 A1) enables a safe atraumatic advancement of the device and in particular of the medical catheter within the body lumen. Furthermore, the computational demand when advancing the device and in particular the medical catheter can be reduced because merely the positioning of the distal end of the device has to be optimized when advancing the device.

The system can comprise a visualization device, in particular a display, for displaying the position and a partial or the complete shape of the device relative to a three-dimensional shape of the body lumen.

Thus, the position and shape of the device and in particular the medical catheter, relative to the three-dimensional shape of the body lumen, can be monitored, e.g. by a clinician.

The advancement actuator can be configured for advancing the device with a speed of at least 1.5 cm, in particular through a heart valve opening, in less than 0.7 s, in particular less than 0.5 s, preferably in less than 0.3 s.

By configuring the advancement actuator for rapid advancement of the device along the longitudinal direction of the device, the device can be advanced through a heart valve opening within a fraction of a cardiac cycle. This can prevent contact with the heart valve during, advancement of the device and allows for synchronized advancement of the device by the advancement actuator with respect to the cardiac cycle controlled by the processing unit.

The advancement actuator can comprise at least one driving roller, optionally with at least one non-driven roller, which frictionally engage the device for advancing the device based on an input from the processing unit.

The advancement actuator can additionally or alternatively comprise at least one pinching (e.g. finger-like) element, in particular a plurality of pinching elements, for frictionally engaging the device and advancing/retracting the device in accordance with a translational and non-rotational movement of the pinching element. Based on an input from the processing unit the pinching element of the advancement actuator can frictionally engage and be advanced/retracted jointly with the device.

The invention is now described with reference to certain embodiments and figures which show:
- Figure 1:: a schematic representation of a system comprising a first embodiment of a medical device and a medical catheter introduced into the heart of a patient;
- Figure 2:: a cross-section of a second embodiment of the medical device showing an enlarged view of a distal portion of the medical device;
- Figure 3:: a cross-section of a third embodiment of the medical device deploying a mounted heart valve prosthesis;
- Figures 4A and 4B:: arrangements of an ultrasound probe which may be used with the medical device of Figs. 1 - 3;
- Figure 5A:: a system comprising a medical device with an ultrasound probe according to Figs. 4A and 4B and a medical catheter;

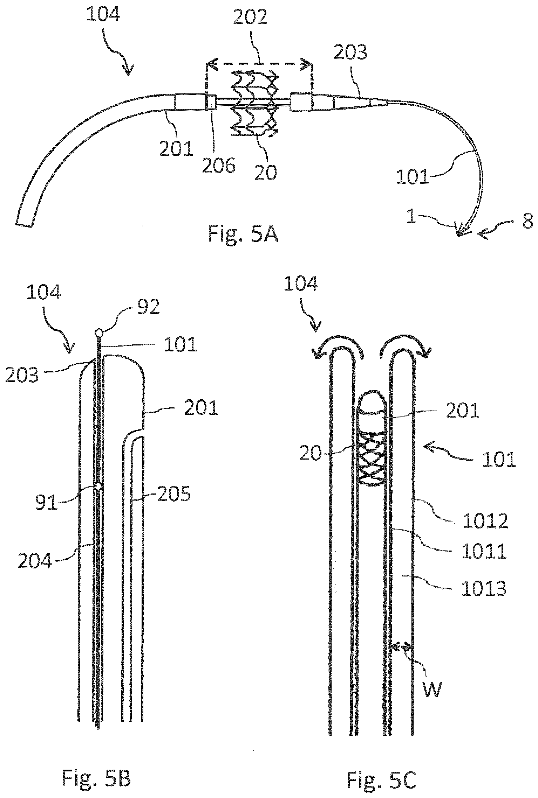

- Figures 5A - 5C:: three systems comprising different embodiments of a medical device and a medical catheter;

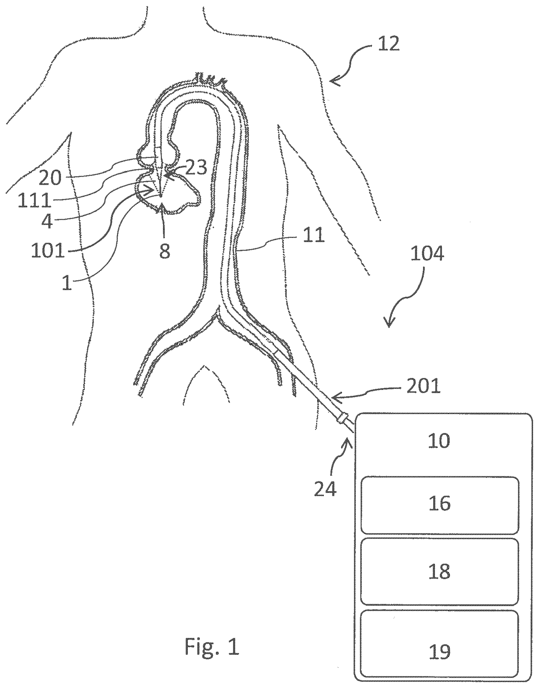

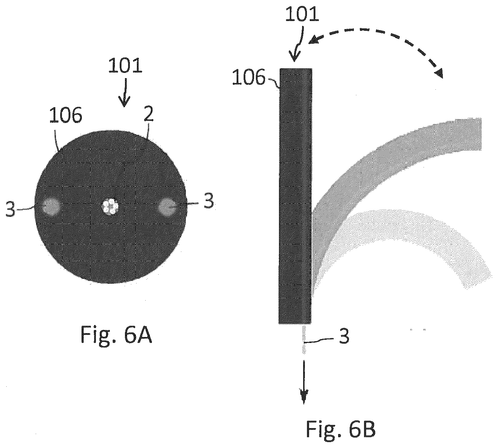

- Figures 6A and 6B:: a schematic cross-section and longitudinal cross-section of a medical device, e.g. a medical device in Figs. 1 - 3 and Figs. 5A - 5B;

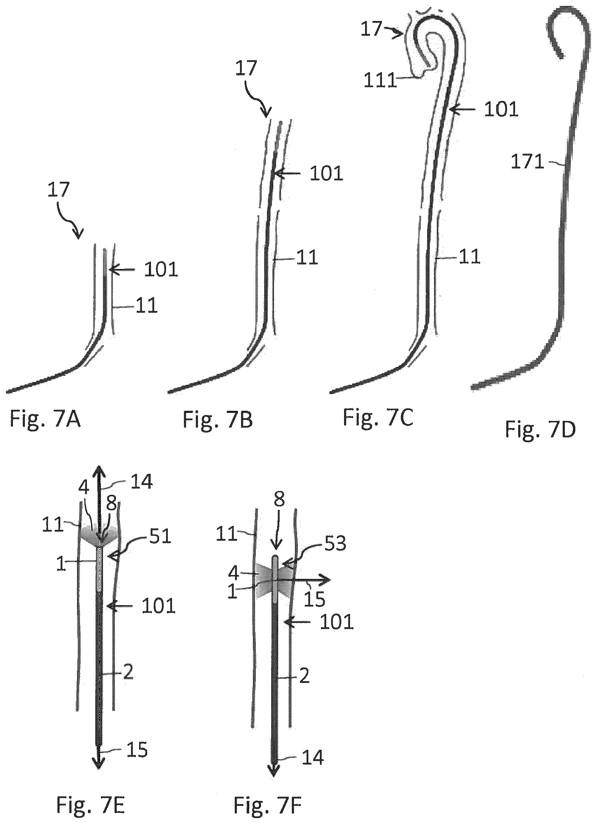

- Figures 7A - 7C:: a schematic reconstruction of a three-dimensional model obtained by advancing the medical device along an aorta of a patient;
- Figure 7D:: a schematic model of a shape of a medical device obtained by advancing the medical device along an aorta of a patient;
- Figures 7E and 7F:: a medical device with an ultrasound probe in a distally-looking configuration and a medical device which has an ultrasound probe in a sidewardly-looking configuration.

Figure 1 shows a system 104 with a medical device 101 and a medical catheter 201 which are introduced through a femoral artery and through the aorta 11 of a patient 12 up to the aortic valve annulus 111. The medical device 101 is arranged within a lumen of the catheter 201 between a proximal end 24 of the medical catheter 201 and a distal end 23 of the medical catheter 201 which extends across the aortic valve annulus 111. The medical device 101 has an outer diameter of 1.5 mm such that the medical device 101 can be used like a guidewire and inserted through the aortic valve annulus 111 into the left ventricle with minimal risk and within a fraction of a cardiac cycle. The medical device 101 is operatively connected to an extracorporeally arranged processing unit 10 which is configured for operating the medical device 101 fully automated based on a reinforcement learning algorithm or deep learning algorithm. The processing unit 10 is connected to an advancement actuator 16 comprising a driven and non-driven roller or a translationally movable pinching/pushing element (not shown in Fig. 1) for an axial movement of the medical device.101 within the lumen of the medical catheter 201 or within the vessel of the patient 12. The processing unit 10 is further connected to a display 18 to provide visual feedback to a surgeon and as a quality control measure. The processing unit 10 is also connected via an interface to a solid-state data storage 19 which provides pre-operative volume data acquired by a computer tomography scan of the aorta 11 and aortic valve annulus 111 acquired prior to the surgical procedure. The interface between the processing unit 10 and the data storage 19 allows for transmitting between the processing unit and the data storage 19 and storing data on the data storage 19.

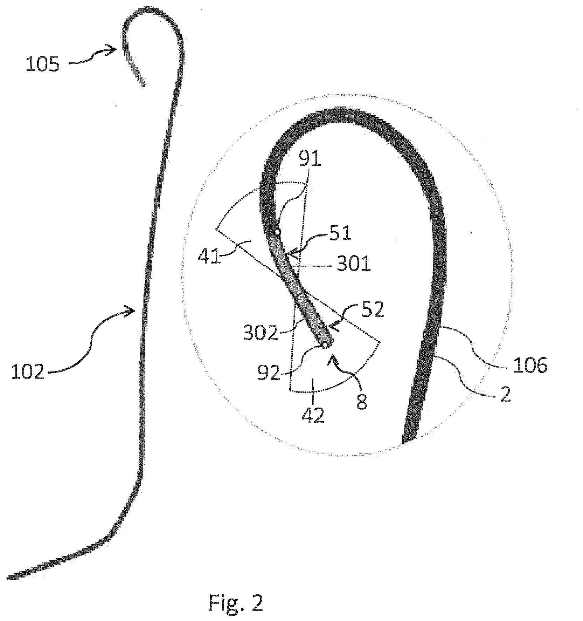

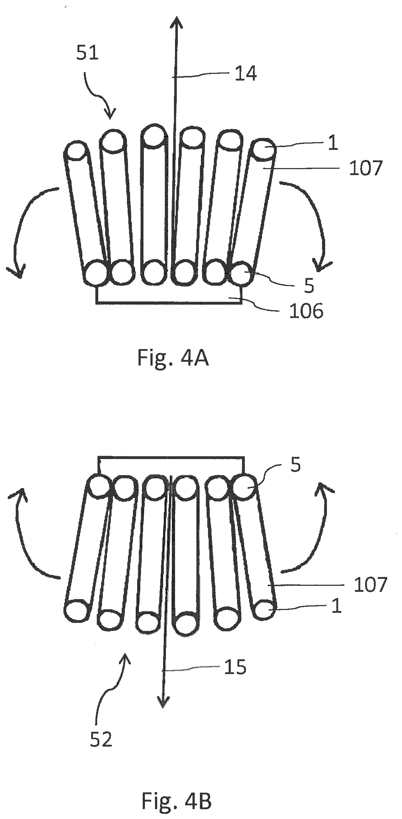

In Fig. 1 the medical device 101 comprises an ultrasound probe 1 located at its distal end 8. A field of view 4 of the ultrasound probe 1 is orientable by a positioning unit (not shown in Fig. 1) along a proximal direction of the medical device 101 and is movable at an angular range of 180° between a distal direction and the proximal direction. The field of view 4 has an angulation of 45° around the proximal direction. Alternatively multiple ultrasound probes 1 can be located in a particular orientation at the distal end (see e.g. Fig. 2) and/or multiple ultrasound probes 1 can be orientable by a positioning unit (see e.g. Fig. 4). The placement of the device 101 within the left ventricle in Fig. 1 allows local imaging of the aortic valve annulus 111 from a side opposite the distal end of the catheter 201 with the ultrasound probe 1. The local images allow identification of calcifications and stenoses/obstructions via the processing unit 10 that are difficult to identify from a proximal side of the aortic valve annulus 111 and/or the valves. The processing unit 10 is adapted to reconstruct a local blood flow based on the local images using the Doppler effect. The processing unit 10 is further adapted to reconstruct a three-dimensional blood flow map based on the local blood flow. The processing unit may not necessarily be one integrally formed unit but may be divided into separate processing elements, in particular comprising a cloud-based data processing element.

Based on the local images the processing unit 10 is further adapted to calculate a reconstructed three-dimensional model (see Fig. 7A - 7C) of the aorta, the heart valve annulus 111 and the leaflets/ventricle of the heart, which is augmented with the shape of the device 101 (see Fig. 7D). The processing unit 10 is further configured to register and adjust the three-dimensional model with the pre-operative computer tomography stored on the storage 19. The processing unit 10 is further adapted to include data concerning the patient specific cardiac cycle and corresponding deformations of the aorta 11 and the heart valve annulus 111 in the reconstructed model.

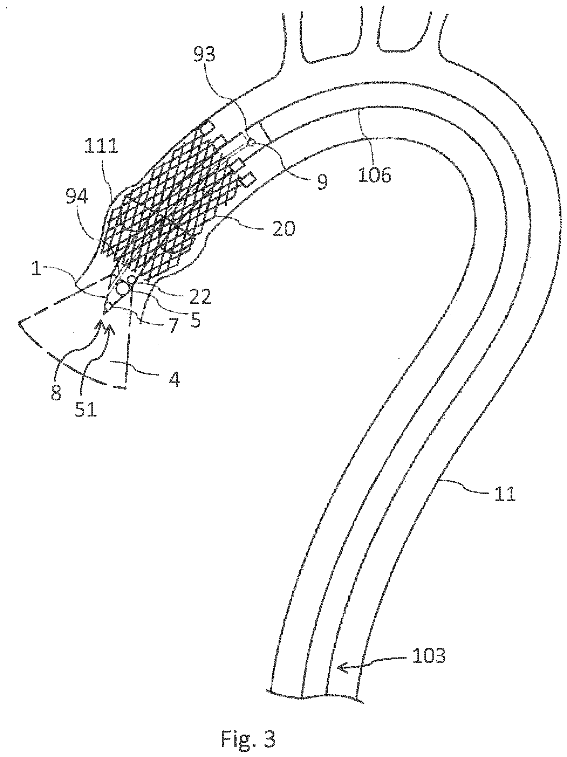

The processing unit 10 is further configured to control the advancement of the medical catheter 201 along the medical instrument 101 into the aortic valve annulus 111 based on the reconstructed model. Prior to advancement, the processing unit 10 is further configured for displaying the reconstructed three-dimensional model on the display 18 showing the position and shape of the medical device 101 with respect to the aorta 11 and valve annulus 111. The processing unit 10 is adapted to adjust the three-dimensional model to overlay the model with a virtual model of the heart valve prosthesis, accounting for tissue properties of the valve annulus 111. Thereby, a heart valve prosthesis 20 (see Fig. 3) which is mounted on within a deployment section 202 of the medical catheter 201 can be deployed for implantation, or in particular replacement of an existing heart valve prosthesis.

After the implantation of the heart valve prosthesis, the processing unit is adapted to verify a position of the prosthetic heart valve with respect to the aortic valve annulus 111. The positioning of the heart valve prosthesis can be verified based on processed data stored on the storage 19 by the processing unit 10, e.g. the pre-operative computer tomography data, the reconstructed three-dimensional model, the reconstructed blood flow, a pressure value and/or based on proximally-looking local images of the ultrasound probe 1 acquired after implantation.

Figure 2 shows a cross-section of a second embodiment of a medical device 101 showing an enlarged view of a distal portion 105 of the medical device 101. The medical device 101 has an elongated body 106 made from a biocompatible plastic. Centrically arranged within the elongated body 106 is a shape sensing unit 2 which comprises a plurality of optical fibers with fiber Bragg gratings which extend over the entire length of the medical device 101 (see e.g. US 9,404,734 B2). An extracorporeally arranged processing unit (not shown in Fig. 2) at a proximal end of the device 101 is configured to reconstruct the three-dimensional shape of the medical device 101. The shape of the medical device 101 can be reconstructed based on a light input controlled by the processing unit into the plurality of optical fibers. The shape can be reconstructed based on the resulting reflected light measured by the processing unit based on the detected wavelengths and corresponding intensities of the reflected light. The processing unit is further adapted to include the reconstructed shape of the medical device 101 in a three-dimensional model.

The medical device 101 of Fig. 2 has a first ultrasound probe 301 located on a distal end 8 which has a fixed orientation along a first field of view 41 in a proximally-looking configuration 51 with respect to the medical device 101. The medical device 101 further has a second ultrasound probe 302 which is arranged distally to the first ultrasound probe 301 and which is fixedly oriented in a distally-looking configuration 52 of the medical device 101. The transducers of the ultrasound probes 301, 302 are arranged at a curved array resulting in an opening angle of the field of view of 45°.

The medical device 101 further has a first pressure sensor 91 which is arranged on an outwardly facing surface of the medical device 101. The processing unit is connected to the first pressure sensor 91 and configured for measuring the local static pressure via the first pressure sensor 91, such that a longitudinal and a radial position of the pressure sensor 91 with respect to a body lumen can be determined.

-A terminal end of a distal end 8 of the medical device 101 has a second pressure sensor 92 which is connected to the processing unit. The second pressure sensor 92 is adapted for measuring the complete pressure, consisting of the static and dynamic pressure, at the distal end 8 of the device 101. Based on both pressure sensors 91, 92, a pressure gradient between the positions of the pressure sensors 91, 92 can be determined by the processing unit, such that the placement of a prosthetic heart valve can be optimized. The processing unit is adapted to create a three-dimensional pressure map based on the detected pressure values of the pressure sensors 91, 92.

In another alternative embodiment, the medical device 101 has no, or only a forward-looking ultrasound probe 302 and placement of the heart valve prosthesis is carried out solely based on the detected pressure values by the pressure sensors 91, 92.

Figure 3 shows a third embodiment of the medical device 103 forming a medical catheter for placement of a heart valve prosthesis 20. The medical device 103 has an ultrasound probe 1 located on a distal end 8 of the medical device 103. The ultrasound probe 1 is convertible between a distally-looking configuration 51 with a field of view 4 oriented along a distal direction of the device 103 to a proximally-looking configuration with a field of view 4 oriented along a proximal direction of the device 103 by actuating a positioning unit 5 located on the distal end 8. The positioning unit 5 is controllable by a processing unit at a proximal end of the device 103 (not shown in Fig. 3).

A contact force pressure sensor 7 is located at a terminal end of the distal end 8 of the device 103 is configured to detect if the distal end 8 contacts the aorta 11 or a flap of a heart valve.

Furthermore, the distal end 8 of the device 101 comprises a pacing member 22 which is designed to provide rapid pacing during the transcatheter aortic valve implantation when deploying the heart valve prosthesis 20. Fig. 3 shows the partially deployed heart valve prosthesis 20 within the aortic valve annulus 111 during rapid pacing.

Fig. 3 shows that the medical device 103 is introduced through the aorta 11 of a patient up to the aortic valve annulus 111. The device 103 in Fig. 3 has one pressure sensor 9 which is configured for measuring the pressure at the distal end 8 and a circumferential position of the device 103 via two separate channels 93, 94. The first channel 93 extends radially outward from the pressure sensor 9 to a radial opening and thus the pressure sensor 9 can measure a static pressure in the outer region of an elongated body 106 of the device 103. The second channel 94 extends from the pressure sensor 9 in an axial direction of the elongated body 106 to an opening at terminal end of the distal end 8 of the device 103. Therefore, the pressure sensor 9 can measure a total pressure at the distal end via the second channel 94. Measuring the pressure at a position proximal to the heart valve prosthesis 20 and distal to the heart valve prosthesis 20 before and after the deployment of the heart valve prosthesis 20 allows for improved placement and monitoring if an intended placement/positioning of the heart valve prosthesis 20 is achieved.

Figures 4A and 4B show an arrangement of the ultrasound probes 1, which may be used as an alternative to the ultrasound probes of one of the preceding embodiments of the medical devices of Figs. 1 - 3. A plurality of ultrasound probes 1 are arranged on respective arm extensions 107 which are pivotably connected to a positioning unit 5. The positioning unit 5 is actuatable based on a control signal from the processing unit (not shown in Fig. 4) such that the imaging probes 1 are converted from a proximally-looking configuration 51 to a distally-looking configuration 52 or vice versa. The positioning unit 5 is immovably connected to an elongated body 106 of the medical device which is only show schematically in Figs. 4A and 4B. However, the positioning unit 5 may alternatively be attached to a distally facing surface e.g. at a terminal end of the distal end of the medical device.

While converting the ultrasound probes 1 between the proximally-looking and distally-looking configurations 51, 52, the ultrasound probes 1 can continuously acquire local images, thereby carrying out a volume scan in a specific angular range. Since the extension arms 107 allow the imaging probes 1 to be positioned closer to a wall of a body lumen, e.g. a wall of the left ventricle of a heart, a local image quality can be increased.

Furthermore, the processing unit is configured to actuate the positioning unit 5 to carry out the volume scan by simultaneously moving all imaging probes 1 in unison by tilting them relative to a longitudinal axis of the medical device along a proximal or distal direction 15, 14. Based on the acquired local images of the ultrasound probes 1, the processing unit is configured to derive a virtual model of the three-dimensional shape, e.g. of the left ventricle. Thus, a detailed three-dimensional model, for example of the left ventricle, can be reconstructed via the processing unit. Moreover, a cardiac cycle of a patient is per default taken into account since the local images of the plurality of ultrasound probes 1 are acquired simultaneously at the respective angles of the arm extensions 107 with respect to the axial direction of the medical device.

Figures 5A shows a system 104 comprising a medical device 101 comprising an arrangement of ultrasound probes 1 according to Figs. 4A and 4B and a medical catheter 102. The medical device 101 in Fig. 5A is insertible through a lumen of a medical catheter 201 and the arrangement of ultrasound probes 1 is arranged on a distal end 8 of the medical device 101. The medical catheter 201 has a conical distal end 203 for insertion into a left ventricle of a heart of a patient. The medical catheter 201 further has a deployment section 202 which can be slidably opened by moving a sleeve 206 surrounding a heart valve prosthesis 20 of the deployment section in an axial direction of the medical catheter 201 to deploy the self-expanding heart valve prosthesis 20.

Figure 5B shows a longitudinal cross-section of a system 104 comprising a medical device 101 which is inserted into a first tubular lumen 204 of a medical catheter 201. The first lumen 204 leads from a proximal end of the medical catheter 202 up to an opening at a distal end 203 of the medical catheter 201. The medical device 101 has two pressures sensors 91, 92 which are spaced apart from each other in a longitudinal direction of the medical device 101. The medical device 101 is advanceable by an advancement actuator (not shown in Fig. 5B) with respect to the medical catheter 202. The medical catheter 201 in Fig. 5B further has a second tubular lumen 205 leading from the proximal end to a radial opening. The medical device 101 or a second medical device is insertable into the second lumen 205, e.g. for introduction into a branching lumen of the aorta, like a coronary artery. Additionaly or alternatively, the second lumen 205 or a plurality of additional lumen can be used for a pressure measurement, e.g. by extending to a pressure sensor at a proximal end of the device 101 or by leading to a pressure sensor 9 analogous to the channels 93, 94 in Fig. 3.

Figure 5C shows a longitudinal cross-section of a system 104 comprising a medical device 101 which has an inner portion 1011 and an outer portion 1012 and a medical catheter 201. The inner portion 1011 is evertible to the outer portion 1012 by applying a pressure to the spacing 1013 between the inner and outer portion 1011, 1012 with a width W of 1 mm. Figure 5C further shows that the medical catheter 201 carrying a heart valve prosthesis 20 on an outer surface is arranged concentrically surrounded by the medical device 101 (see e.g. WO 2022/057296 or EP 2 1315 286 A1 for the evertible medical device).

Figures 6A and 6B show a schematic cross-section and a longitudinal cross-section of a medical device 101, e.g. a medical device of Figs. 1 - 3, 5A, or 5B. The medical device 101 has a tubular elongated body 106 which has three lumens. Fig. 6A shows that the centric lumen has a plurality of optical fibers 2 with fiber Bragg gratings arranged therein such that a processing unit can reconstruct the three-dimensional shape of the medical device 101. The two opposing lumens are arranged radially outward within the tubular body 106 and pull wires 3 or push rods are movably arranged within the lumens.

Fig. 6B further shows that one of the pull wires 3 can be actuated to deflect the curvature of the elongated body 106 of the device 101. The processing unit is configured for fully automated deflection of the elongated body 106 by actuation of the pull wires 3 such that the medical device 101 does not contact a body lumen of a patient. The deflection controlled by the processing unit is based on the reconstructed three-dimensional shape of the medical device 101 measured via the optical fiber 2 and the reconstructed three-dimensional model of the aorta, heart valve annulus and ventricle.

Figures 7A to 7C show a schematic reconstruction of a three-dimensional model 17 obtained by advancing the medical device 101 along an aorta 11 up to an aortic valve annulus 111 carried out by a processing unit 10 (see Fig. 1). The medical device 101 has an imaging probe 1 at a distal end 8 which has a field of view 4 which can be directed in a proximal direction 15 of the device 101 analogous to the medical device of one of the embodiments Fig. 1 - Fig. 3 or Fig. 5A (not shown in Fig 7A - 7F).

Furthermore, the medical device 101 has an ultrasound probe 1 either in a distally-looking configuration 51 (see Fig. 7E), in a sidewardly-looking configuration 53 (see Fig. 7F) or a combination of both. Fig.'7E shows that a field of view 4 of the ultrasound probe 1 in the distally-looking configuration is oriented in the in a distal direction 14 of the medical device 101. Fig. 7F shows that the field of view 4 of the ultrasound probe 1 is oriented in a radial direction 16 of the medical device 101 such that the field of view is essentially perpendicular to a wall of the aorta 11.

Figures 7A to 7C further show that the reconstructed three-dimensional model 17 is continuously augmented/completed fully automated via the processing unit when advancing the medical device 101 further within the aorta 11. In addition, the three-dimensional model includes the reconstructed shape 171 of the medical device 2 measured via the optical fibres 2 having fiber Bragg gratings which is exemplary shown in Fig. 7D. The three-dimensional model further includes the relative position of the reconstructed shape 171 of the medical device 101 with respect to the reconstructed three-dimensional shape of the aorta 11.

The reconstructed three-dimensional model 17 is augmented to a four-dimensional model by including temporal-dependent structural deformations of the aorta 11, the valve annulus 111 and in particular the valve leaflets, based on the cardiac cycle of a patient (not shown in Figs. 7A - 7F). Moreover, the three-dimensional model is registered to three-dimensional pre-operative computer tomography data based on specific landmarks identified within the processed data via the processing unit. Augmenting the reconstructed three-dimensional model with the pre-operational data allows for a more detailed and complete mapping of the aorta 11, valve annulus 111 and a left ventricle and in particular to identify spatial positions of calcifications.

The three-dimensional model is augmented via the processing unit by a feature extraction which can be carried out using keypoint detection mechanisms such as scale-invariant feature transform (SIFT) in combination with random sample consensus (RANSAC), ellipse fitting, and/or deep learning convolutional neural networks such as U-Net, transformers, and/or holistically nested edge detection (HED).

## Claims

1. A medical device (101, 103) for use with or as a part of an elongated medical catheter (201), preferably during transcatheter valve implantation, the device (101, 103) comprising
at least one imaging probe (1), in particular an ultrasound probe, for local imaging of a body lumen (11) of a patient (12) and
a shape sensing unit (2) for detecting a partial or complete shape (13) of the device (101, 103),
wherein the imaging probe (1) is arranged neighbouring a distal end of the device (101, 103) and in such a way that a field of view (4) of the imaging probe (1) is or can be arranged such as to image an area proximal to the distal end, preferably by the field of view (4) being directed in a proximal direction (15) of the device (101, 103).

2. Device (101, 103) according to claim 1, wherein the device (101, 103) is insertable into the elongated medical catheter (201), into the body lumen (11) or a branching body lumen (11) or wherein
the device (101, 103) is advanceable over the elongated medical catheter (201), in particular by the device (101, 103) being evertible in a longitudinal direction of the elongated medical catheter (201).

3. Device (101, 103) according to any one of the preceding claims, wherein the device (101, 103) comprises a deflecting mechanism (3) for partially of fully deforming the device (101, 103) and in particular the elongated medical catheter (201).

4. Device (101, 103) according to one of the preceding claims, wherein the device (101, 103) comprises a plurality of imaging probes (1), wherein the field of view (4) of one of the imaging probes (1) is preferably oriented in a distal direction (14) and the field of view (4) of one of the imaging probes (1) is oriented in a proximal direction (15) of the device (101, 103).

5. Device (101, 103) according to one of the preceding claims, wherein the device (101, 103) comprises at least one positioning unit (5) which is adapted
- (i) for displacing the at least one imaging probe (1) along a circumferential direction of the device (101, 103) and/or for rotating the imaging probe (1) such as to tilt its field of view (4) relative to a longitudinal axis of the device (101, 103) and/or
- (ii) for rotating a plurality of imaging probes (1) such as to tilt the field of view (4) relative to the longitudinal axis,
such that the at least one imaging probe (1) is reversibly movable from a distally-looking configuration (51) having a field of view (4) oriented in the distal direction (14) to a proximally-looking configuration (52) having a field of view (4) oriented in the proximal direction (15) of the device (101, 103).

6. Device (101, 103) according to one of the preceding claims, wherein the device (101, 103) comprises a contact force sensor (7) neighbouring the distal end (8) of the device (101, 103).

7. Device (101, 103) according to one of the preceding claims, wherein the device (101, 103) comprises at least one pressure sensor (9) for measuring a local pressure of the body lumen (11) external to the device (101, 103), in particular at least two pressure sensors (91, 92) which are arranged spaced apart, in particular spaced apart in the longitudinal direction of the device (101, 103).

8. Device (101, 103) according to claim 7, wherein the device (101, 103) comprises at least one channel (91) leading from a measurement site to the pressure sensor (9), in particular at least two channels (91) leading from a respective measurement site to a respective pressure sensor (9) or a common pressure sensor (9).

9. Device (101, 103) according to claim 7 or 8, wherein the at least one pressure sensor (9) is configured for measuring the static pressure at a circumferential section of the device (101, 103) and/or the total pressure consisting of a dynamic and the static pressure at a front side of the distal end (8) of the device (101, 103).

10. Device (101, 103) according to one of the preceding claims, wherein the device (101, 103) comprises at least one sensing element which is selected from the group of a conductivity sensor and a bio-impedance sensor.

11. A device (102), optionally a device (101, 103) according to one of the preceding claims, in particular for use with an elongated medical catheter (201) during transcatheter valve implantation, comprising
at least one imaging probe (1), in particular an ultrasound probe, for local imaging of a body lumen (11) of a patient (12) and
a shape sensing unit (2) for detecting a partial or complete shape (13) of the device (102),
**characterized in that** the device (102) comprises at least two pressure sensors (91, 92) which are spaced apart from each other or movable relative to each other, in particular in a longitudinal direction of the device (102), the pressure sensors (91, 92) being configured for detecting a pressure gradient within the body lumen (11).

12. A system (104) comprising a medical device (101, 102, 103), preferably according to one of the claims 1 to 10, in particular for use with an elongated medical catheter (201) during transcatheter valve implantation,
the device (101, 102, 103) having a shape sensing unit (2) for detecting a partial or complete shape (13) of the device (101, 102, 103) and at least one of:
(i) at least one imaging probe (1), in particular an ultrasound probe, for local imaging of a body lumen (11) of a patient (12), which is arranged neighbouring a distal end (8) of the device, a field of view (4) of the imaging probe (1) being arranged or arrangeable in a proximal direction (15) of the device (101) and
(ii) multiple spaced apart pressure sensors (91, 92) located on the device (101, 102, 103) or insertable into the device (101, 102, 103),
wherein the system (104) further has a processing unit (10) connected to or connectable to the shape sensing unit (2) and the imaging probe (1) and/or the pressure sensors (91, 92) for processed data relating to at least one of:
- a local image of the body lumen (11) acquired by the imaging probe (1), in particular acquired in a distally-looking configuration (51) having a field of view (4) oriented in a distal direction (14) of the imaging probe (1) and/or
acquired by the imaging probe (1) in a proximally-looking configuration having a field of view (4) oriented in the proximal direction (15),
- the partial or complete shape (13) of the device (101, 102, 103) acquired by the shape sensing unit (2),
- patient-specific pre-operative three-dimensional data, in particular pre-operative computer tomography data,
- a detected signal of a contact force sensor (7), and
- a detected signal of the pressure sensors (91, 92),
the processing unit (10) being further configured to determine, based on the processed data, at least one operating signal for operating at least one of a deflecting mechanism (3) of the device (101, 102, 103), a positioning unit (5) for positioning the imaging probe (1), and an advancement actuator (16) for advancing the device (101, 102, 103).

13. System (104) according to claim 12, wherein the processing unit (10) is configured to carry out a volume scan by operating the positioning unit (5) to move the imaging probe (1) from the distally-looking configuration (51) to the proximally-looking configuration (52) or vice versa and reconstruct or augment a three-dimensional model (17) based on the volume scan, in particular a model of a vascular network of a left ventricle of a heart,
and wherein the processing unit (10) is further configured to determine, based on the three-dimensional model (17), at least one operating signal for operating at least one of the deflecting mechanism (3) of the device (101, 102, 103), the positioning unit (5), and the advancement actuator (16) for advancing the device (101, 102, 103).

14. System (104) according to one of the claims 12 or 13, wherein the processing unit (10) is configured to process the local images of the imaging probe (1) to determine a blood flow in a local section of the body lumen (11).

15. System (104) according to one of the claims 12 to 14, wherein the processing unit (10) is adapted to overlay a virtual model of a prosthetic heart valve with a virtual three-dimensional model of an area of a patient, preferably the reconstructed three-dimensional model (17), in particular by using landmarks in the reconstructed three-dimensional model or by using a machine learning algorithm based on the reconstructed three-dimensional model (17), in particular accounting for tissue properties,
the processing unit (10) further being adapted to display the overlayed virtual models on a display (18).

16. System (104) according to one of the claims 12 to 15, wherein the processing unit (10) is adapted to register images, in particular the local images of the proximally-looking configuration (52) and/or the reconstructed three-dimensional model (17), with the pre-operative three-dimensional data, in particular to identify calcification locations based on the pre-operative three-dimensional data.

17. System (104) according to one of the claims 12 to 16, wherein the system (104) comprises a memory unit (19) connected or connectable to the processing, unit (10) for storing data of at least one of:
- the pre-operative volume data (21),
- the reconstructed three-dimensional model (17) or the overlayed virtual models,
- local blood flow,
- the detected signal of the contact force sensor (7), and
- the detected signal of the at least one pressure sensor (9) .

18. System (104) according to claim 17, wherein the processing unit (10) is adapted for verifying a position of a prosthetic heart valve (20) within the heart based on at least one of the stored data of the memory unit (19) prior to a placement of a prosthetic heart valve (20) by comparison to processed data after the placement of the prosthetic heart valve (20).

19. System (104) according to one of the claims 12 to 18, wherein the processing unit (10) is adapted to operate the deflecting mechanism (3) and/or the advancement actuator (16) based on the processed data and in particular on at least one of the stored data, such that the distal end (8) of the device (101, 102, 103), in particular the complete device (101, 102, 103), preferably the medical catheter (201) remains centred within the body lumen (11) at least partially along its length.

20. System (104) according to one of the claims 12 to 19, wherein the processing unit (10) is adapted for operating the device (101, 102, 103) during a transcatheter valve implantation in a fully automated manner, in particular based on a reinforcement learning algorithm.

21. System (104) according to one of the claims 12 to 20, wherein the system (104), in particular the device (101, 102, 103), comprises a pacing member (22) for rapid pacing.

22. System (104) according to one of the claims 12 to 21, wherein the processing unit (10) is adapted to adjust the partial or complete shape (13) of the device (101) via the deflecting mechanism (3) when advancing the device (101) in the body lumen (11) via the advancement actuator (16) based on the spatial position and orientation of the distal end (8) of the device (101) with respect to the body lumen (11) such that subsequent portions of the device (101) adopt the same spatial position and orientation with respect to the body lumen (11).

23. System (104) according to one of the claims 12 to 22, wherein the system (104) comprises a visualization device, in particular a display (18), for displaying the position and the partial or complete shape (13) of the device (101) relative to a three-dimensional shape of the body lumen (11) .

24. System (104) according to one of the claims 12 to 23, wherein the advancement actuator (16) is configured for advancing the device (101) at least 1.5 cm, in particular through a heart valve opening, in less than 0.7 s, in particular less than 0.5 s, preferably in less than 0.3 s.
